# EUROPEAN PATENT APPLICATION

(11) **EP 2 886 536 A1**
(43) Date of publication of application: **24.06.2015**
(21) Application number: 13198613.5
(22) Date of filing: 19.12.2013
(51) Int. Cl.: C07D 235/16, A61K 31/4184

(54) **Bendamustine derivatives as anti-proliferative agents**

(71) Applicant: Schickaneder, Helmut, 90542 Eckental (DE)
(72) Inventor: Schickaneder, Helmut, 90542 Eckental (DE)
(74) Representative: Danner, Stefan

(57) **Abstract**

The present invention relates to bendamustine derivatives and related compounds as well as their medical application.

## Description

### Field of the invention

The present invention relates to bendamustine derivatives and related compounds as well as their medical application.

### Background of the invention

Bendamustine (IUPAC name: 4-{5-[bis(2-chloroethyl)amino]-1-methyl-1H-benzimidazol-2-yl}butanoic acid) having the structural formula 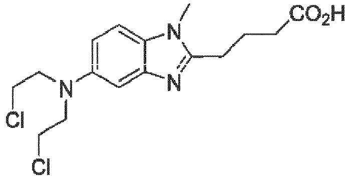 is a nitrogen mustard belonging to the family of drugs called alkylating agents. Bendamustine has been shown to be effective in the treatment of chronic lymphocytic leukemias and lymphomas. Bendamustine is normally used in its hydrochloride salt form as active agent. However, efficacy in terms of cytotoxicity and/or cytostaticity is a challenging issue and a critical problem.

Conventionally, bendamustine (HCl) is prepared by a process as disclosed, e.g., in DD 34727 and J. Chen et al., Org. Process Res. Dev. 2011, 15, p. 1063-1072. Furthermore, DD 34727 discloses derivatives of bendamustine hydrochloride having the following structural formulas 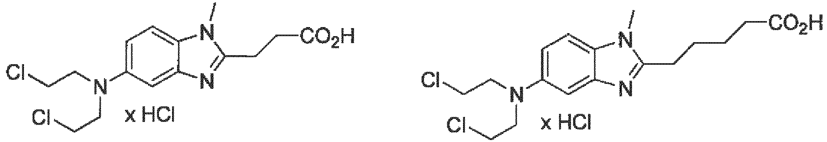 in which derivatives the carbon chain linking the carboxylic acid moiety to the benzimidazole ring structure is shortened by one methylene unit and extended by one methylene unit, respectively. However, anti-proliferative activities of the aforementioned higher and the lower homologs of bendamustine have not been reported.

There is still a need for bendamustine derivatives, and thus an object of the present invention is to provide bendamustine related compounds with useful properties and therapeutic effects, and therapeutic uses thereof.

### Summary of the invention

The object is solved by a compound of formula IX according to claim 1, a compound of formula VIII according to claim 4, a compound of formula VII according to claim 5, processes according to claim 10 and 13 and a pharmaceutical composition according to claim 14. Preferred embodiments are set forth below as well as the dependent ciaims.

Various aspects, advantageous features and preferred embodiments of the present invention as summarized in the following items, respectively alone or in combination, contribute to solve the object of the invention:
(1) A compound of formula IX or a salt thereof 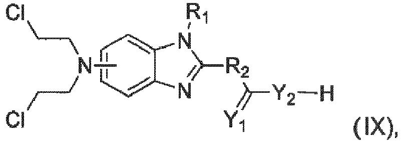 wherein the bis(2-chloroethyl)amino group is attached to position 6 of the benzimidazole ring structure; and
   wherein R₁ is alkyl, aryl or alkylaryl; R₂ is alkanediyl, arylene, alkylarylene or arylalkanediyl; and Y₁ and Y₂ are independently from each other oxygen or sulfur.

In a specific embodiment, the present invention relates to a compound of formula IX, wherein R₂ is alkanediyl provided that the alkanediyl moiety does not represent mono-hydroxylated propandiyl.

The terms "alkyl" and "alkanediyl", as used herein, denotes straight, branched or cyclic hydrocarbons having a typical meaning, preferably 1 to 12 carbon atoms, preferably 1 to 8 carbon atoms, and more preferably 1 to 6 carbon atoms.

The term "aryl(ene)", as used herein, denotes hydrocarbon aryls having a typical meaning, preferably 3 to 12 carbon atoms, preferably single or condensed six-membered rings, more preferably phenyl.

The terms "alkylaryl(ene)" and "arylalkane(diyl)", as used herein, denotes that the aforementioned aryl(ene) moieties are incorporated into the aforementioned straight or branched alkyl or alkanediyl moieties either at one of the proximal or distal ends of the alkyl or alkanediyl chain or between the alkyl or alkanediyl chains. For example, for R₁, proximal end means adjacent to the nitrogen atom of the benzimidazole ring of compound of formula IX, while distal means the terminal carbon of the alkyl or aryl moiety which is furthermost from said nitrogen atom. For R₂ proximal end means adjacent to -CY₁- of the -CY₁-Y₂- carboxylic acid group of compound of formula IX, while distal means the terminal carbon of the alkyl or alkanediyl moiety which is furthermost from said -CY₁- moiety.

An exemplary compound of formula IX is shown in formula IX':

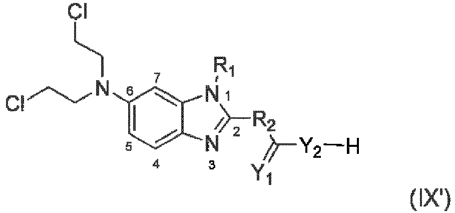

Preferably, the salt of compound of formula IX represents a pharmaceutically acceptable salt.
(2) The compound of formula IX according to item (1), wherein R₁ represents C₁-C₃ alkyl, R₂ represents C₁-C₃ alkanediyl, Y₁ and Y₂ represent oxygen.

As to the meaning of the terms "alkyl", "alkanediyl", reference is made to the explanations under item (1) above.
(3) The compound of formula IX according to item (1) or (2), wherein R₁ is methyl, R₂ is propanediyl, and Y₁ and Y₂ represent oxygen.

The compound defined in item (3) has the structural formula

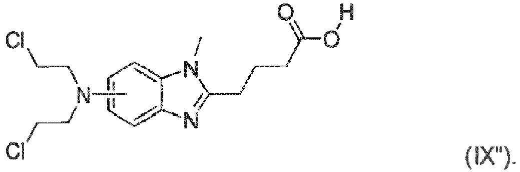

In the following, compounds of formula IX having substituents R₁, R₂, Y₁ and Y₂ as defined in item (3) are named "iso-X-bendamustines", wherein X denotes the position of the N-lost moiety (bis(2-chloroethyl)amino group) at the benzimidazole ring structure of compound of formula IX, i.e. denotes the 6-position. A compound of formula IX" in which the N-lost moiety is attached to the 6-position of the benzimidazole ring structure represents "6-iso-bendamustine".

In a specific embodiment, the present invention relates to a compound of formula IX provided that the compound does not have the structure of formula IX".

The term "conventional bendamustine", as used in the following, means bendamustine having the structural formula 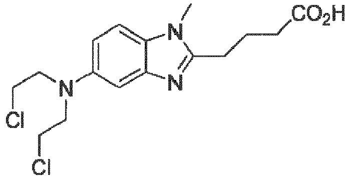 wherein the N-lost moiety is in 5-position of the benzimidazole ring structure.
(4) The compound according to any one of items (1) to (3), wherein the compound of formula IX is in the form of a base addition salt, in which the base is selected from the group consisting of magnesium hydroxide, calcium hydroxide, zinc hydroxide, potassium hydroxide, sodium hydroxide, potassium carbonate, sodium carbonate, potassium hydrogen carbonate, sodium hydrogen carbonate, diethylamine, triethylamine, ethanolamine (2-aminoethanol), diethanolamine (2,2'-iminobis(ethanol)), triethanolamine (2,2',2"-nitrilotris(ethanol)), ethylenediamine, piperazine, piperidine, pyrrolidine, pyridine, morpholine, 1H-imidazole, N-methylglucamine, L-lysine, choline, L-arginine, benethamine, 4-(2-hydroxyethyl)-morpholine, tromethamine, 2-(dimethylamino)ethanol (Deanol), 1-(2-hydroxyethyl)-pyrrolidine, 2-(diethylamino)-ethanol, benzathine, hydrabamine and betaine; or the compound of formula IX is in the form of an acid addition salt according to item (17).
(5) The compound according to any one of items (1) to (4), wherein the compound of formula IX is in the form of a salt, preferably a salt comprising 0.6 to 1.4 mol water relative to 1 mol of compound of formula IX, more preferably 0.8 to 1.2 mol water, even more preferably the salt comprising water is in the form of a hydrate.

The phrase "comprising water", as used herein, denotes that the compound of formula IX comprises the above indicated amounts of water despite of (extensive) drying (e.g. by means of drying under vacuum and/or under heating). This water may be simply adsorbed to the molecule, or it may be incorporated into the crystal lattice of it.

The term "hydrate", as used herein, specifies a crystalline solid adduct, wherein a stoichiometric or nonstoichiometric amount of water is incorporated in the crystal lattice of said crystalline solid. The hydrate of compound of formula IX may be free of organic solvent, but optionally may additionally have organic solvent(s) incorporated.

Preferably, a crystalline pharmaceutically acceptable salt of compound of formula IX"' 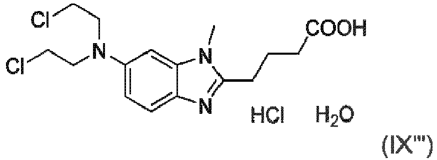 represents 6-iso-bendamustine hydrochloride, more preferably this compound represents a hydrate. Even more preferably, said compound of formula IX"' exhibits a X-ray diffraction (XRD) pattern having at least signals at the following 2θ values (± 0.5° respectively):

| | | | | | | | | | | |
|---|---|---|---|---|---|---|---|---|---|---|
| **2theta [°]** | 7.83 | 10.01 | 13.18 | 13.75 | 14.96 | 15.34 | 18.47 | 22.27 | 24.63 | 26.53 |

(6) A compound of formula VIII or a salt thereof 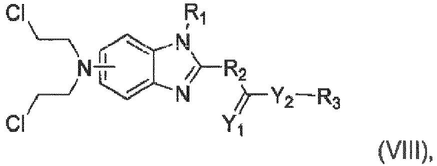 wherein the bis(2-chloroethyl)amino group is attached to position 6 of the benzimidazole ring structure;
   wherein R₁ and R₃ are independently from each other alkyl, aryl or alkylaryl; R₂ is alkanediyl, arylene, alkylarylene or arylalkanediyl; Y₁ and Y₂ are independently from each other oxygen or sulfur; and
   wherein optionally R₃ is substituted with an amine moiety -NR₅R₆ in which R₅ and R₆ independently from each other are a substituted or unsubstituted alkyl, or R₅ and R₆ together represent a C₃-C₇ alkyl chain forming a 4 to 8-membered ring structure together with the nitrogen located between R₅ and R₆, wherein one or more carbon atoms in said ring structure are optionally replaced by heteroatoms selected from the group consisting of nitrogen, oxygen or sulfur.

The meaning of the terms "alkyl", "alkanediyl", "aryl(ene)", "alkylaryl(ene)", and "arylalkane(diyl)" corresponds to the explanations made under item (1) above.

In a specific embodiment, the present invention relates to a compound of formula VIII provided that R₃ does not represent a substituted or unsubstituted cyclic imide.

Preferably, the optional amine substituent -NR₅R₆ is located at the distal end of R₃ which is furthermost from Y₂ of the -CY₁-Y₂- moiety. The following structural formula VIII' 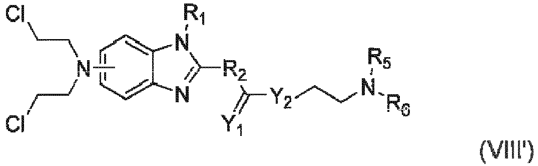 shows an example where R₃ represents ethylene, and the amine moiety -NR₅R₆ is located at the carbon of the ethylene moiety which is furthermost from Y₂ of the -CY₁-Y₂- moiety. In other examples, instead of ethylene, R₃ represents a moiety being selected from the group consisting of alkanediyl, arylene or alkylarylene or arylalkanediyl.

Preferably, the salt of compound of formula VIII represents a pharmaceutically acceptable salt.
(7) A compound of formula VII or an addition salt thereof 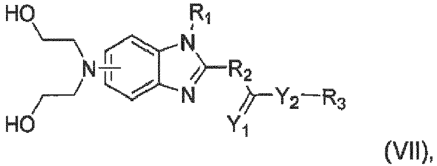 wherein the bis(2-chloroethyl)amino group is attached to position 6 of the benzimidazole ring structure;
   wherein R₁ and R₃ are independently from each other alkyl, aryl or alkylaryl; R₂ is alkanediyl, arylene, alkylarylene or arylalkanediyl; Y₁ and Y₂ are independently from each other oxygen or sulfur; and
   whwerein optionally R₃ is substituted with an amine moiety -NR₅R₆ in which R₅ and R₆ independently from each other are a substituted or unsubstituted alkyl, or R₅ and R₆ together represent a C₃-C₇ alkyl chain forming a 4 to 8-membered ring structure together with the nitrogen located between R₅ and R₆, wherein one or more carbon atoms in said ring structure are optionally replaced by heteroatoms selected from the group consisting of nitrogen, oxygen or sulfur.

The meaning of the terms "alkyl", "alkanediyl", "aryl(ene)", "alkylaryl(ene)", and "arylalkane(diyl)" corresponds to the explanations made under item (1) above.

In a specific embodiment, the present invention relates to a compound of formula VII provided that R₃ does not represent a substituted or unsubstituted cyclic imide.
(8) The compound according to item (6) or (7), wherein R₃ is substituted with an amine substituent -NR₅R₆, in which R₅ and R₆ independently from each other represent C₁-C₄ alkyl or form a 5 to 7-membered ring structure together with the nitrogen located between R₅ and R₆.
(9) The compound according to any one of items (6) to (8), wherein R₅ and R₆ are the same and represent C₁-C₃ alkyl or R₄ and R₅ form a 5 to 7-membered ring structure together with the nitrogen located between R₅ and R₆.
(10) The compound according to any one of items (6) to (9), wherein in the ring structure formed by R₅ and R₆ together with the nitrogen located between R₅ and R₆, one carbon atom is replaced by one nitrogen atom or one oxygen atom, preferably one oxygen atom.
(11) The compound according to item (10), wherein in the ring structure formed by R₅ and R₆, a further nitrogen atom is substituted (-NR₇-) or unsubstituted (-NH-), preferably substituted with R₇ selected from the group consisting of alkyl, aryl, alkylaryl or arylalkyl, more preferably with alkyl.

As to the meaning of the terms "alkyl", "aryl", "alkylaryl" or "arylalkyl", reference is made to the explanations under item (1) above.
(12) The compound according to any one of items (6) to (11), wherein the ring structure formed by R₅ and R₆ together with the nitrogen located between R₅ and R₆ is in the form of a 5 or 6-membered ring, preferably a 6-membered ring.
(13) The compound according to any one of items (6) to (12), wherein the ring structure formed by R₅ and R₆ together with the nitrogen located between R₅ and R₆ is selected from the group consisting of pyrrolidine, piperidine, piperazine and morpholino, preferably piperazine or morpholino, more preferably morpholino.
(14) The compound according to any one of items (6) to (13), wherein the atoms of the ring structure formed by R₅ and R₆ together with the nitrogen located between R₅ and R₆ may be unsubstituted, or substituted with a substituent selected from the group consisting of C₁-C₄ alkyl, C₁-C₄ alkoxy, C₁-C₄ alkylsulfide, unsubstituted amino (-NH₂), dialkylamino in which alkyl is C₁-C₄ alkyl; preferably C₁-C₄ alkyl or C₁-C₄ alkoxy; more preferably the ring structure is unsubstituted.
(15) The compound of formula VII or VIII according to any one of items (6) to (14), wherein R₁ is C₁-C₃ alkyl, R₂ is C₁-C₃ alkanediyl, R₃ is C₁-C₃ alkyl, and Y₁ and Y₂ represent oxygen.
(16) The compound of formula VII or VIII according to any one of items (6), (7) and (15), wherein R₁ is methyl, R₂ is propanediyl, R₃ is ethyl, Y₁ and Y₂ represent oxygen; optionally R₃ is substituted with -NR₅R₆ in which R₅ and R₆ form a morpholino moiety together with the nitrogen located between R₅ and R₆.

The compound of formula VIII defined in item (16) in which R₃ is substituted with -NR₅R₆ in the form of a morpholino moiety has the structural formula 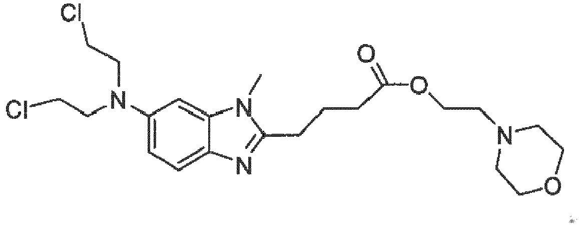
(17) The compounds of formula according to any one items (1) to (16), wherein the compound of formula IX, VIII or VII is in the form of an acid addition salt in which the acid is selected from the group consisting of hydrochloric acid, hydrobromic acid, sulfuric acid, phosphoric acid, glutamic acid, (+)-L-tartaric acid, citric acid, (-)-L-malic acid, DL-lactic acid, L-ascorbic acid, succinic acid, adipic acid, acetic acid, stearic acid, carbonic acid, thiocyanic acid, glycerol-phosphoric acid, L-aspartic acid, maleic acid, fumaric acid, galactaric acid, D-glucuronic acid, glycolic acid, D-glucoheptonic acid, hippuric acid, D-gluconic acid, glutaric acid, sebacic acid, capric (decanoic) acid, lauric acid, palmitic acid, alginic acid, benzoic acid, nicotinic acid, propionic acid, caprylic (octanoic) acid, naphthalene-l,5-disulfonic acid, ethane-l,2-disulfonic acid, cyclamic acid, p-toluenesulfonic acid, methanesulfonic acid, dodecylsulfuric acid, naphthalene-2-sulfonic acid, benzenesulfonic acid, oxalic acid, 2-hydroxy ethanesulfonic acid, ethanesulfonic acid, pannoic (embonic) acid, 2-oxoglutaric acid, 1-hydroxy-2-naphthoic acid, malonic acid, gentisic acid, lactobionic acid, (-)-L-pyroglutamic acid, oleic acid, (+)-camphoric acid, isobutyric acid and orotic acid.
(18) The compounds of formula IX and VIII according to any one of items (1) to (6) and (8) to (17) being in the form of their free acid/base or a pharmaceutically acceptable salt thereof for use in the therapeutic treatment of diseases selected from the group consisting of acute T cell leukaemia, erythroleukemia, Ewing osteosarcoma, (hormone dependent) mamma carcinoma, cervix carcinoma, colorectal cancer, medulloblastoma, glioblastoma and astrocytoma, malignant melanoma, histocytic lymphoma, pancreatic carcinoma, prostate cancer (metastasis of a subclavicular lymph node), large cell bronchial carcinoma, colorectal adenocarcinoma, and osteosarcoma; wherein the disease is preferably selected from the group consisting of acute T cell leukaemia, erythroleukemia, Ewing osteosarcoma, malignant melanoma, histocytic lymphoma, pancreatic carcinoma, prostate cancer (metastasis of a subclavicular lymph node), large cell bronchial carcinoma, colorectal adenocarcinoma, and osteosarcoma; and wherein the disease is more preferably selected from the group consisting of Ewing osteosarcoma, malignant melanoma, pancreatic carcinoma, prostate cancer (metastasis of a subclavicular lymph node), colorectal adenocarcinoma, and osteosarcoma.
(19) A process for preparing a compound of formula V or an acid addition salt thereof, 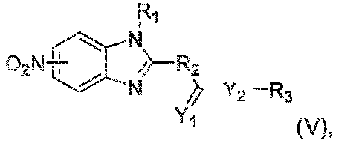 wherein the nitro group is attached to position 6 of the benzimidazole ring structure; and
   wherein R₁ is alkyl, aryl or alkylaryl; R₂ is alkanediyl, arylene, alkylarylene or arylalkanediyl; R₃ is H, alkyl, aryl or alkylaryl; and Y₁ and Y₂ independently from each other are oxygen or sulfur;
   the process comprising:
   (i) when R₃ is alkyl, aryl or alkylaryl, reacting a compound of formula II 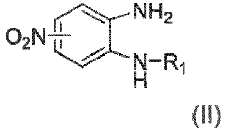 wherein R₁ is defined as above, and the nitro group is attached to any one of positions 3, 4 and 6 of the aniline moiety,
      with a compound of formula III¹ 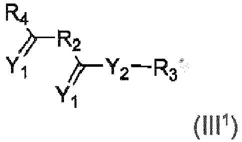 wherein R₂, Y₁ and Y₂ are defined as above, R₃' is alkyl, aryl or alkylaryl, and R₄ is selected from the group consisting of -Y₂-H, -Cl and -Y₂-CY₁-R₂-CY₁-Y₂-R₃'; or
   (ii) when R₃ represents H, reacting a compound of formula II with a compound of formula III² or a compound of formula III³ 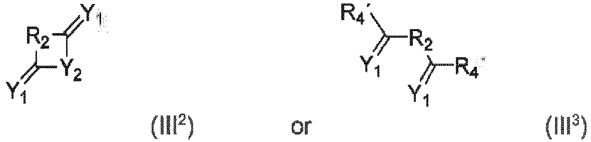 wherein R₂, Y₁ and Y₂ are defined as above, and the two substituents R₄' in formula III³ independently from each other are -Y₂-H or -Cl, preferably both R₄' represent -Y₂-H or one R₄' = -Y₂-H and the other R₄' = -Cl,
      with the proviso that in the case compound of formula III³ with both R₄' = -Cl, the chloro group not forming an amide with the amine moiety of formula II is hydrolized for converting said chloro group to a hydroxy group.

As to the meaning of the terms "alkyl", "aryl", "alkylaryl" or "arylalkyl", reference is made to the explanations under item (1) above. As regards the position of the nitro group at the benzimidazole ring of compound of formula (V), the explanations made for the bis(2-chloroethyl)amino group of compound of formula IX under item (1) likewise apply.

In compound of formula II, the nitro group is attached to any one of positions 3, 4 and 6 of the aniline moiety, as indicated in the following exemplary structural formula II' 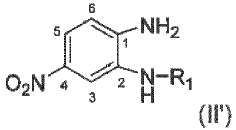 wherein the nitro group is in 4-position. Preferably, the nitro group is located at the 4 position of the aniline moiety of compound of formula II'.
(20) The process according to item (19), wherein said process is carried out without isolation of an intermediate compound of formula IV or an acid addition salt thereof 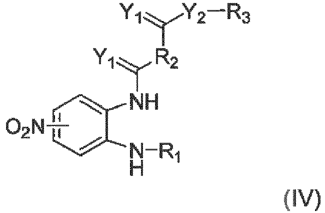 wherein R₁ represents alkyl, aryl or alkylaryl; R₂ represents alkanediyl, arylene, alkylarylene or arylalkanediyl; and R₃ represents H, alkyl, aryl or alkylaryl; Y₁ and Y₂ independently from each other represent oxygen or sulfur.
(21) The process according to item (19) or (20), wherein R₁ is C₁-C₃ alkyl, R₂ is C₁-C₃ alkanediyl, R₃ is H or C₁-C₃ alkyl, and Y₁ and Y₂ represent oxygen.
(22) The process according to any one of items (19) to (21), wherein R₁ is methyl, R₂ is propanediyl, R₄ is H or ethyl and Y₁ and Y₂ represent oxygen, preferably R₁ is methyl, R₂ is propanediyl, R₃ is ethyl and Y₁ and Y₂ represent oxygen.
(23) The process according to any one of items (19) to (22), wherein in the compound of formula III¹, III² and III³, Y₁ and Y₂ represent oxygen; and/or R₄ = -Y₂-CY₁-R₂-CY₁-Y₂-R₃'.

Preferably, compound of formula III² is glutaric anhydride having the structural formula 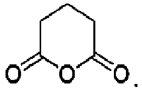
(24) The process according to any one of items (19) to (23), wherein the compound of formula V' or an acid addition salt thereof 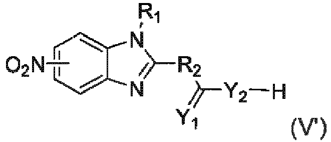 wherein R₁ represents alkyl, aryl or alkylaryl; R₂ represents alkanediyl, arylene, alkylarylene or arylalkanediyl; Y₁ and Y₂ independently from each other represent oxygen or sulfur, wherein the nitro is attached to position 6 of the benzimidazole ring structure,
   is further converted to compound of formula V" or an acid addition salt thereof 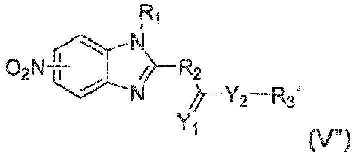 wherein R₁, R₂, Y₁, Y₂ and the position of the nitro group are defined as above, and R₃' represents alkyl, aryl or alkylaryl,
   by means of transesterification with an alcohol of formula R₃'-OH, wherein R₃' is defined as above.
(25) The process according to item (24), wherein transesterification is carried out in the presence of a proton donor, preferably an inorganic proton donor, more preferably an inorganic proton donor selected from the group consisting of hydrohalic (hydrohalogeneic) acid and sulfuric acid (H₂SO₄), even more preferably HCl or H₂SO₄, in particular H₂SO₄.

The term "proton donor", as used herein, means a Bronsted acid which provides for donating proton(s).
(26) The process according to item (24) or (25), wherein R₃' is C₁-C₃ alkyl, preferably ethyl.
(27) A process for preparing a compound of formula IX, VIII or VII according to any one of items (1) to (17) comprising the steps of:
   (a) preparing a compound of formula V according to a process of any one of items (19) to (26), and
   (b) further converting compound of formula V to any one of compounds of formula IX, VIII or VII.

The meaning of the terms "alkyl", "alkanediyl", "aryl(ene)", "alkylaryl(ene)", and "arylalkane(diyl)" corresponds to the explanations made under item (1) above.
(28) A pharmaceutical composition comprising compound of formula IX and/or VIII according to any one of items (1) to (6) and (8) to (17) in the form of their free acid/base or a pharmaceutically acceptable salt thereof as a pharmaceutically active agent(s) and at least one pharmaceutically acceptable excipient.

The term "pharmaceutically active agent", as used herein, means any active pharmaceutical ingredient intended for treatment or prophylaxis of a disease of a subject to be treated, specifically mammals such as humans. In general it means any active pharmaceutical ingredient that has an effect on the physiological conditions of the subject.

The term "pharmaceutically acceptable excipient", as used herein, means any physiologically harmless or inert, pharmacologically inactive material compatible with the physical and chemical characteristics of the active agent. Suitable pharmaceutically acceptable excipients are generally known in the art.
(29) The pharmaceutical composition according to item (28) for oral and/or parenteral administration.

### Detailed description of the invention

The present invention will be described in the following with respect to particular embodiments and with reference to certain drawings but the invention is to be understood as not limited thereto but only by the appended claims. The drawings described are only schematic and are to be considered non-limiting.

Where the term "comprising" is used in the present description and claims, it does not exclude other elements or steps. For the purposes of the present invention, the term "consisting of" is considered to be a preferred embodiment of the term "comprising". If hereinafter a group is defined to comprise at least a certain number of embodiments, this is also to be understood to disclose a group, which preferably consists only of these embodiments.

Where an indefinite or definite article is used when referring to a singular noun e.g. "a", "an" or "the", this includes a plural of that noun unless specifically stated otherwise.

In case, numerical values are indicated in the context of the present invention the skilled person will understand that the technical effect of the feature in question is ensured within an interval of accuracy, which typically encompasses a deviation of the numerical value given of ± 10%, and preferably of ± 5%.

Furthermore, the terms first, second, third, (a), (b), (c), and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Further definitions of term will be given in the following in the context of which the terms are used. The following terms or definitions are provided solely to aid in the understanding of the invention. These definitions should not be construed to have a scope less than understood by a person of ordinary skill in the art.

According to one aspect of the invention, highly desirable compounds derived from bendamustine analogues are provided in the form of compound of formula IX or a salt thereof 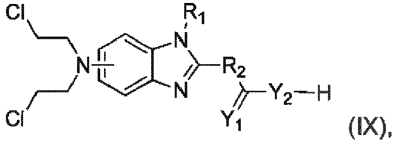 wherein the bis(2-chloroethyl)amino group is attached to position 6 of the benzimidazole ring structure; and
wherein R₁ is alkyl, aryl or alkylaryl; R₂ is alkanediyl, arylene, alkylarylene or arylalkanediyl; and Y₁ and Y₂ are independently from each other oxygen or sulfur.

It was surprisingly found by the present invention that the novel 6 positions of the N-lost moiety in the isobendamustine derivatives of compound of formula IX provide for a beneficial alteration of both electronic as well as steric effects compared to conventional bendamustine in which the N-lost moiety is in 5-position of the benzimidazole ring structure. Without wishing to be bound to theory, it is believed that these altered electronic and/or steric effects provide for a good or even an improved antiproliferative potency of compound of formula IX compared to conventional bendamustine (HC!). Furthermore, antiproliferative potency and further useful properties may be additionally fine-tuned and eventuaiiy improved by suitable selections for substituents R₁, R₂, Y₁ and Y₂.

According to another aspect of the invention, compounds of formula VII or VIII or (pharmaceutically acceptable) acid addition salts thereof, are provided 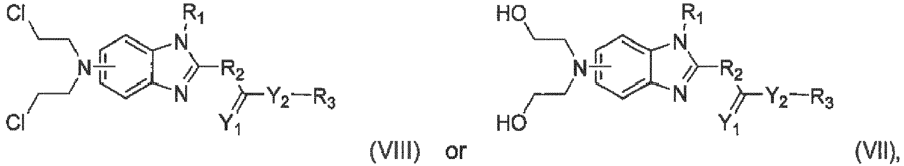 wherein the bis(2-chloroethyl)amino group is attached to position 6 of the benzimidazole ring structure;
wherein R₁ and R₃ are independently from each other alkyl, aryl or alkylaryl; R₂ is alkanediyl, arylene, alkylarylene or arylalkanediyl; Y₁ and Y₂ are independently from each other oxygen or sulfur; and
wherein optionally R₃ is substituted with an amine moiety -NR₅R₆ in which R₅ and R₆ independently from each other are a substituted or unsubstituted alkyl, or R₅ and R₆ together represent a C₃-C₇ alkyl chain forming a 4 to 8-membered ring structure together with the nitrogen located between R₅ and R₆, wherein one or more carbon atoms in said ring structure are optionally replaced by heteroatoms selected from the group consisting of nitrogen, oxygen or sulfur.

As regards compounds of formula VIII, without wishing to be bound to theory, it is believed that these compounds exhibit a good or even an improved antiproliferative potency compared to conventional bendamustine. In particular, it is believed that the ester compounds of formula VIII provide for an improved cellular uptake compared with bendamustine in form of the free acid owing to an improved combination of solubility in aqueous medium and increased passage through cell membranes compared with bendamustine (HCl). Hence, compounds of formula VIII represent valuable active pharmaceutical ingredients with a better administration (oral, parenteral).

Compounds of formula VII represent valuable intermediate compounds for preparing active pharmaceutical ingredients such as iso-bendamustine derivatives.

According to a preferred embodiment, any one of compounds of formulae IX, VIII and VII may be advantageously structurally modified by structural modifications (a) to (d), respectively alone or in combination:
(a) R₁ is C₁-C₆ alkyl, R₂ is C₁-C₆ alkanediyl, and Y₁ and Y₂ are oxygen; wherein preferably R₁ is C₁-C₄ alkyl, R₂ is C₁-C₄ alkanediyl, and Y₁ and Y₂ are oxygen;
(b) R₁ is C₁-C₃ alkyl, R₂ is C₁-C₃ alkanediyl, and Y₁ and Y₂ are oxygen;
(c) R₁ is methyl, R₂ is propanediyl, and Y₁ and Y₂ are oxygen; and
(d) in compounds of formula VIII and VII, R₃ is C₁-C₃ alkyl, and preferably ethyl.

According to a further preferred embodiment, in compounds of formulae VIII and VII, R₃ is substituted with an amine moiety -NR₅R₆. Preferably, said amine moiety is in the form of a ring structure formed by R₅ and R₆ together with the nitrogen located between R₅ and R₆ has at least one of the following structural characteristics (i) to (v), respectively alone or in combination:
(i) one carbon atom is replaced by one nitrogen atom or one oxygen atom, preferably by one oxygen atom;
(ii) a further nitrogen atom is substituted (-NR₇-) or unsubstituted (-NH-), preferably substituted with R₇ being selected from the group consisting of alkyl, aryl, alkylaryl or arylalkyl, more preferably with alkyl;
(iii) the ring structure is in the form of a 5 or 6-membered ring, preferably a 6-membered ring;
(iv) the ring structure is selected from the group consisting of pyrrolidine, piperidine, piperazine and morpholino, preferably being piperazine or morpholino, and more preferably being morpholino;
(v) the atoms of the ring structure are unsubstituted, or substituted with a substituent selected from the group consisting of C₁-C₄ alkyl, C₁-C₄ alkoxy, C₁-C₄ alkylsulfide, unsubstituted amino (-NH₂), dialkylamino in which alkyl is C₁-C₄ alkyl; the substituent preferably being C₁-C₄ alkyl or C₁-C₄ alkoxy; and more preferably the ring structure being unsubstituted.

Without wishing to be bound to theory, it is believed that structural modifications (i) to (v) provide for particularly advantageous modifications in terms of solubility in aqueous solution and efficacy in terms of cytotoxicity.

According to a particular embodiment of the present invention, compounds of formulae VIII and IX, being in the form of their free acid/base or a pharmaceutically acceptable salt thereof, are used as a medicament for the therapeutic treatment of diseases selected from the group consisting of acute T cell leukaemia (Jurkat, T B-152), erythroleukemia (HEL 92.1.7, TIB-180), Ewing osteosarcoma (SK-ES1, HTB-86), (hormone dependent) mamma carcinoma (MCF-7, HTB-22), cervix carcinoma (multidrug resistant KB-V1), colorectal cancer, medulloblastoma (Daoy, HTB-186), glioblastoma (U-118MG, HTB-15; LN-18, CRL-2610) and astrocytoma (SW1783, HTB-13), malignant melanoma (SK-Mel3, HTB-69), histocytic lymphoma (U-937; CRL-1593.2), pancreatic carcinoma (Capan-1, HTB-80), prostate cancer (metastasis of a subclavicular lymph node) (LnCap clone FGC, CRL-1740), large cell bronchial carcinoma (NCI-H460, HTB-177), colorectal adenocarcinoma (HT-29, HTB-38), osteosarcoma (MG-63, CRL-1427), wherein the acronyms in brackets designate the cell line and the corresponding ATCC-number representative of the respective cancer entities.

As an example, the synthesis of 6-iso-bendamustine isomer of formula IX"' starting from compound of formula VII" is illustrated in the following Scheme 1:

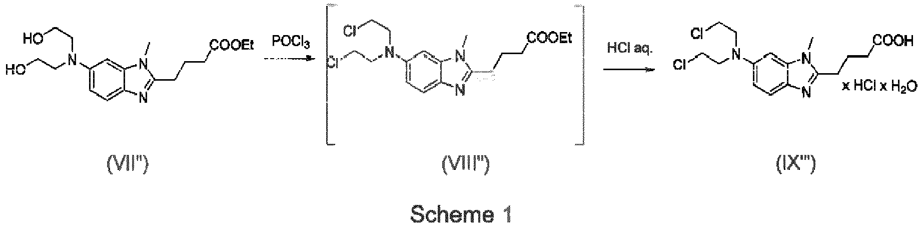

As can be gathered from Scheme 1, the compound of formula VII" may be converted to compound of formula VIII" by means of chlorination, using e.g. phosphorous oxychloride as chlorinating agent, and in a subsequent step, compound of formula VIII" is subjected to ester cleavage by means of e.g. aqueous HCl in order to obtain 6-isobendamustine hydrochloride hydrate of compound of formula IX"'.

The precursor compound of formula VII" can be efficiently prepared, for example by means of the synthetic pathway starting from the readily available starting material 4-nitrobenzene-1,2-diamine (compound of formula I", as depicted in Scheme 2 below: 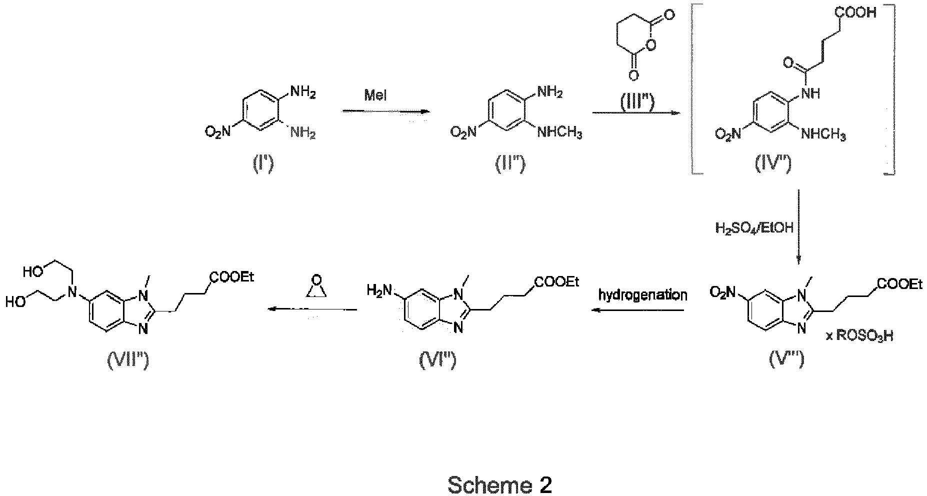 According to another aspect of the invention, a process is provided for preparing a compound of formula V or an acid addition salt thereof 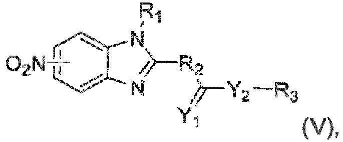 wherein the nitro group is attached to position 6 of the benzimidazole ring structure; and wherein R₁ is alkyl, aryl or alkylaryl; R₂ is alkanediyl, arylene, alkylarylene or arylalkanediyl; R₃ is H, alkyl, aryl or alkylaryl; and Y₁ and Y₂ independently from each other are oxygen or sulfur; the process comprising:
(i) when R₃ is alkyl, aryl or alkylaryl, reacting a compound of formula II 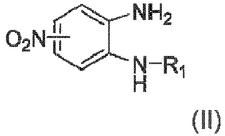 wherein R₁ is defined as above, and the nitro group is attached to any one of positions 3, 4 and 6 of the aniline moiety, with a compound of formula III¹ 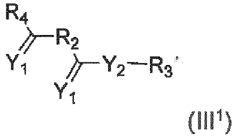 wherein R₂, Y₁ and Y₂ are defined as above, R₃' is alkyl, aryl or alkylaryl, and R₄ is selected from the group consisting of -Y₂-H, -Cl and -Y₂-CY₁-R₂-CY₁-Y₂-R₃'; or
(ii) when R₃ represents H, reacting a compound of formula II with a compound of formula III² or a compound of formula III³ 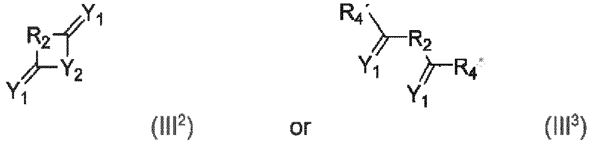 wherein R₂, Y₁ and Y₂ are defined as above, and the two substituents R₄' in formula III³ independently from each other are -Y₂-H or -Cl, preferably both R₄' represent -Y₂-H or one R₄' = -Y₂-H and the other R₄' = -Cl,
   with the proviso that in the case compound of formula III³ with both R₄' = -Cl, the chloro group not forming an amide with the amine moiety of formula II is hydrolized for converting said chloro group to a hydroxy group.

It was surprisingly found by the inventors that compound of formula II readily forms an imidazole structure of compound of formula V when it is reacted with a compound of formula III¹, III² or III³. In particular, the nitro group in 3, 4 and 6 position of compound of formula II provides for a significantly improved reactivity of the amino group of compound of formula II compared to e.g. N²-methyl-4-nitro-1,2-diamine having the structural formula (in which the positions of the benzene ring are indicated with the respective numbers) 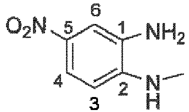 which is typically applied as the starting material for synthesizing the benzimidazole moiety of conventional bendamustine. However, when reacting N²-methyl-4-nitro-1,2-diamine with glutaric anhydride, due to the relative poor reactivity of N²-methyl-4-nitro-1,2-diamine, no benzimidazole ring structure is formed, rather, an amide compound having the structural formula 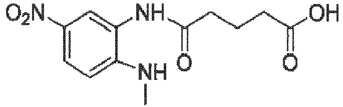 is isolated, as can be gathered e.g. from prior art documents DD 34727 and J. Chen et al., Org. Process Res. Dev. 2011, 15, p. 1063-1072. In contrast to the aforementioned prior art process, the present process renders possible to dispense with isolation of an intermediate amide compound, rather, owing to the improved reactivity of compound of formula II due to electronic and/or steric effects imparted by the nitro group in 3, 4 and 6 position of the aniline moiety, imidazole compound of formula V can be directly obtained. This finding suggests that - compared to conventional bendamustine - electronic as well as steric effects will become significant also in terms of pharmacological activity and optionally further properties of a compound of formula IX prepared from compound of formula V.

According to yet another aspect of the present invention, a process for preparing a compound of formula IX, VIII or VII according to any one of items (1) to (10) is provided, which process comprises the steps of:
(a) preparing a compound of formula V according to a process of any one of items (12) to (19), and
(b) further converting compound of formula V to any one of compounds of formula IX, VIII or VII.

As regards this aspect of the invention, owing to the introduction of a substituent convertible to a N-lost group (bis(2-chloroethyl)amino) to position 6 of the benzimidazole ring structure of compound of formula V at an early stage of the synthetic pathway, subsequent reaction steps for converting compound of formula V to a bendamustine isomer of compound of formula IX can be carried out analogously to reaction steps known in prior art for the preparation of conventional bendamustine. That is, said subsequent reaction steps do not require laborious modifications of reaction conditions and/or reaction pathway.

According to another aspect of the invention, a pharmaceutical composition is provided which comprises compound of formula IX and/or compound of formula VIII in the form of their free acid/base or a pharmaceutically acceptable salt thereof as a pharmaceutically active agent(s) and at least one pharmaceutically acceptable excipient.

Preferably, suitable pharmaceutically acceptable excipient(s) is/are selected from the group consisting of binders, disintegrants, bulk polymers, glidants, lubricants and preservatives, without being limited thereto.

The term "binder", as used herein, denotes a binding agent which improves adhesion in between particles of the pharmaceutically active agent.

The term "disintegrant", as used herein, denotes an agent providing for rapid disintegration of a pharmaceutical composition into smaller fragments when in contact with water, wherein dissolution of the pharmaceutical composition and, in particular, of a pharmaceutically active agent comprised therein is improved.

The term "bulk polymer", as used herein, denotes a polymeric filling agent, which is typically added to a pharmaceutical composition in suitable amounts.

The term "glidants and lubricants", as used herein, denotes components acting as formulation and processing aids.

The term "preservatives", as used herein, denotes a substance or mixture of substances which prevents decomposition of a pharmaceutical composition, e.g. microbial or bacterial decomposition.

The invention is further described by the the following examples, which are solely for the purpose of illustrating specific embodiments of this invention, and are not to be construed as limiting the claimed subject matter in any way.

### Examples

### Example 1: N²-methyl-4-nitrobenzene-1,2-diamine (compound II')

N²-methyl-4-nitrobenzene-1,2-diamine having the structural formula 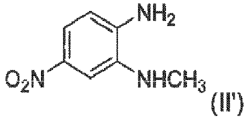 was prepared as follows:
A 500 ml round bottom flask was charged with 4-nitrobenzene-1,2-diamine (40.0 g, 260 mmol), methyl iodide (13 ml, 210 mmol) and DMF (300 ml). Saturated sodium carbonate solution (60 ml) was added with stirring and the mixture was stirred for 18 h.
After filtration the solution was concentrated to dryness in vacuum and the residue was purified by flash column chromatography (ethyl acetate/heptanes = 7:3) to afford N²-methyl-4-nitrobenzene-1,2-diamine in the form of a red oil. Yield: 27.9 g (63 %).

¹H NMR (500 MHz, DMSO-d6): δ = 7.50 (dd, 1H, *⁴J* = 2.8 Hz, *³J* = 8.7 Hz, H-4), 7.10 (d, 1 H, *⁴J*=2.8 Hz, H-6), 6.55 (d, 1 H, *³J* =8.7 Hz, H-3), 6.12 (s, 2H, NH₂), 5.20 (q, 1 H, *³J*= 5.0 Hz, NH), 2.78 (d, 3H, *³J*= 5.0 Hz, CH₃).
¹³C NMR (126 MHz, DMSO-d6): δ=143.6, 137.2, 136.4 (C), 115.6, 110.5, 102.6 (CH), 29.8 (CH₃).
LC-MS (ESI⁻): m/z = 166 (M - H⁺).

### Example 2: Ethyl 4-(1-methyl-6-nitro-1H-benzimidazol-2-yl)butanoic acid (compound V')

Ethyl 4-(1-methyl-6-nitro-1 H-benzimidazol-2-yl)butanoic acid having the structural formula 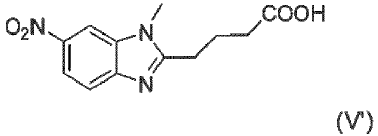 was prepared as follows:

N²-Methyl-4-nitrobenzene-1,2-diamine (9.65 g, 57.7 mmol) was mixed with ethyl acetate (170 ml) and glutaric anhydride (7.47 g, 65.4 mmol) was added. The mixture was heated to reflux for 5.5 h, and a yellow-orange precipitate was observed after 1 h from the orange reaction solution. Then the suspension was cooled on ice for 45 min, followed by vacuum filtration. The collected precipitate was washed with ice-cold EtOAc (3 x 5 ml) and ice-cold EtOH (2 x 5 ml) to yield 6.16 g (41%) of ethyl 4-(1-methyl-6-nitro-1H-benzimidazol-2-yl)butanoic acid in the form of a yellow solid.

¹H-NMR (DMSO): δ = 8.52 (1 H, d, J = 2.2 Hz, Ar), 8.06 (1 H, dd, J = 8.8, 2.2 Hz, Ar), 7.71 (1 H, d, *J* = 8.8 Hz, Ar), 3.84 (3 H, s, CH₃), 2.95 (2 H, t, J = 7.5 Hz, CH₂), 2.39 ( 2 H, t, J = 7.5 Hz, CH₂), 2.02 (2 H, t, J = 7.5 Hz, CH₂).
¹³C-NMR (DMSO): δ = 174.09, 160.67, 146.83, 142.02, 135.13, 118.31, 116.92, 106.87, 32.88, 29.96, 25.97, 21.72.
LC-MS (ESI⁺): m/z = 264.1 (M+H⁺)

### Example 3: Ethyl 4-(1-methyl-6-nitro-1H-benzimidazol-2-yl)butanoate ethyl sulfate (compound V")

Ethyl 4-(1-methyl-6-nitro-1H-benzimidazol-2-yl)butanoate ethyl sulfate having the structural formula 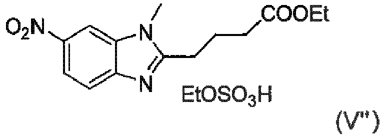 was prepared as follows:
5-{[2-(methylamino)-4-nitrophenyl]amino}-5-oxopentanoic acid (6.06 g, 23.09 mmol) was suspended in ethanol (40 ml) and heated to reflux. After 50 min, H₂SO₄ (1.82 ml, 34.07 mmol) was added over 15 min. The obtained solution was slowly cooled to 0°C. At 10°C, a thick suspension was obtained that was re-warmed to 40-43°C and cooled again to 0°C to get a better stirrable mixture. After stirring 30 min on ice, the suspension was vacuum-filtered and washed with ice-cold ethanol (3 x 5 ml) to yield 6.65 (69%) of ethyl 4-(1-methy)-6-nitro-1H-benzimidazol-2-yl)butanoate ethyl sulfate in the form of an off-white solid.

### Example 4: Ethyl 4-(1-methyl-6-nitro-1 H-benzimidazol-2-yl)butanoate (compound V"')

Ethyl 4-(1-methyl-6-nitro-1H-benzimidazol-2-yl)butanoate having the structural formula 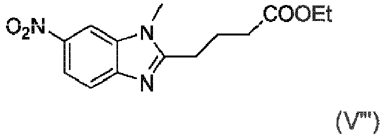 was prepared as follows:
Ethyl 4-(1-methyl-6-nitro-1H-benzimidazol-2-yl)butanoate ethyl sulfate (6.60 g, 15.8 mmol) was suspended in water (330 ml). NaOH (50%, 2.04 g) was diluted with water (10 ml) and added over 10 min. The suspension was stirred for 1 h at r.t. and vacuum-filtered (washed with water (2x10 ml). After drying at 60°C for 1 h, 4.23 g (92%) of free base of ethyl 4-(1-methyl-6-nitro-1H-benzimidazol-2-yl)butanoate was obtained in the form of an off-white powder.

¹H-NMR (DMSO): δ = 8.54 (1 H, d, J = 2.2 Hz, aromatic), 8.07 (1 H, dd, J = 8.9, 2.2 Hz, aromatic), 7.72 (1 H, d, J = 8.9 Hz, aromatic), 4.05 (2 H, q, J = 7.1 Hz, COOCH₂CH₃), 3.85 (3 H, s, CH₃), 2.97 (2 H, t, J = 7.5 Hz, CH₂), 2.49 (2 H, t, J = 7.5 Hz, CH₂), 2.06 (2 H, pent, J = 7.5 Hz, CH₂), 1.17 (3 H, t, J = 7.1 Hz, COOCH₂CH₃).
¹³C-NMR (DMSO): δ = 172.42, 160.47, 146.79, 142.01, 135.11, 118.29, 116.88, 106.85, 59.72, 32.65, 29.93, 25.83, 21.57, 14.00.
LC-MS (ESI⁺): m/z = 292.1 (M+H⁺)

### Example 5: Ethyl 4-(6-amino-1-methyl-1H-benzimidazol-2-yl)butanoate (compound VI")

Ethyl 4-(6-amino-1-methyl-1 H-benzimidazol-2-yl)butanoate having the structural formula 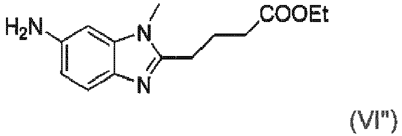 was prepared as follows:
A hydrogenation reactor was charged with ethyl 4-(1-methyl-6-nitro-1 H-benzimidazol-2-yl) butanoate (4.2 g, 14.4 mmol) and ethanol (300 ml). After complete dissolution and
inertization of the vessel, Pd on activated charcoal catalyst (5%, moist, 340 mg) was added and hydrogenation (4.5 bar, 60°C) was performed for 4 h (no further H₂ consumption). The catalyst was removed by filtration and after evaporation of the solvent, 4.0 g of crude ethyl 4-(6-amino-1-methyl-1H-benzimidazol-2-yl)butanoate was obtained and was subsequently recrystallized from ethanol (20 ml). 2.73 g (72.5%) and additional 0.87 g (23%) from the mother liquor of the recrystallization step were obtained after drying in vacuum (40°C).
¹H-NMR (DMSO): δ = 7.18 (1 H, d, J = 8.4 Hz, aromatic), 6.51 (1 H, d, *J* = 1.8 Hz, aromatic), 6.46 (1 H, dd, *J* = 8.4, 1.8 Hz, aromatic), 4.85 (2 H, s, NH₂), 4.05 (2 H, q, *J* = 7.1 Hz, COOCH₂CH₃), 3.54 (3 H, s, CH₃), 2.77 (2 H, t, *J* = 7.4 Hz, CH₂), 2.44 (2 H, t, *J* = 7.4 Hz, CH₂), 1.97 (2 H, pent, *J* = 7.4 Hz, CH₂), 1.17 (3 H, t, *J* = 7.1 Hz, COOCH₂CH₃).
¹³C-NMR (DMSO): δ = 172.57, 151.23, 144.14, 136.79, 134.20, 118.27, 110.36, 93.09, 59.65, 32.76, 28.99, 25.51, 22.10, 14.01.
LC-MS (ESI⁺): m/z = 262.2 (M+H⁺)

### Example 6: Ethyl 4-{6-[bis(2-hydroxyethyl)amino]-1-methyl-1H-benzimidazol-2-yl} butanoate(compound VII")

Ethyl 4-{6-[bis(2-hydroxyethyl)amino]-1-methyl-1 H-benzimidazol-2-yl}butanoate having the structural formula 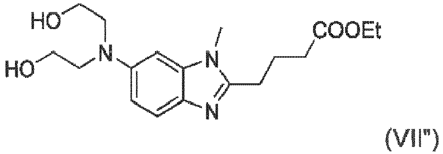 was prepared as follows:

Ethyl 4-(6-amino-1-methyl-1H-benzimidazol-2-yl)butanoate (1.0 g, 3.83 mmol) was dissolved in acetic acid (3.0 ml) and water (8.5 ml). Ethylene oxide gas was added into the solution in intervals for 4 h and stirred at room temperature for additional 15 h. A second feed of oxirane was performed and stirring was continued for another 6 h. The reaction was followed by TLC (10% methanol in DCM). After reaction, the reaction solution was added dropwise into a solution of K₂CO₃ (6 g) in water (20 ml) at 0-5°C. A suspension together with a compact amorphous solid was filtered, and the resulting filter cake was washed with water (2 x 10 ml) and dried overnight at 40°C in a constant stream of air (1.21 g, 90%).

HPLC purity: 95.9% rel. area.
¹H NMR (500 MHz, DMSO-d₆, ppm): δ = 7.30 (d, J = 9.5 Hz, 1H, Arom. CH=CN), 6.63 (d, J = 9.5 Hz, 1H, Arom. CH=CN(CH₂CH₂OH)₂), 6.62 (s, 1H, Arom. CH=CNMe), 4.75 (br s, 2H, OH), 4.05 (q, J = 7.1 Hz, 2H, COOCH₂CH₃), 3.61 (s, 3H, NCH₃), 3.57 (t, J= 6.4 Hz, 4H, 2xCH₂N), 3.44 (t, J= 6.4 Hz, 4H, 2xCH₂O), 2.80 (t, J = 7.4 Hz, CH₂-imidazole), 2.44 (t, J = 7.4 Hz, CH₂COO), 1.9 (p, J = 7.4 Hz, CH₂CH₂CH₂), 1.18 (t, J = 7.1 Hz, COOCH₂CH₃) ¹³C-{H}-NMR (125 MHz, DMSO-d₆, ppm): δ = 172.58 (COO), 151.83 (NC=N), 144.41 (C-N(CH₂CH₂OH)₂), 137.06 (Arom.), 133.83 (Arom.), 118.34 (Arom.), 108.14 (Arom.), 91.68 (Arom. (NMe)CCHCN(CH₂CH₂OH)₂), 59.68 (NCH₂), 58.29 (OCH₂), 54.07 (COOCH₂), 32.76 (probably NCH₃), 29.06 (probably CH₂COO), 25.58 (CH₂-imidazole), 22.12 (CH₂CH₂CH₂), 14.03 (CH₃).
LC-MS (ESI⁺): m/z = 350.1 (M+H⁺)

### Example 7: 6-iso-bendamustine ethylester (IUPAC-nomenclature: ethyl 4-{6-[bis(2-chloroethyl)amino]-1-methyl-1H-benzimidazol-2-yl}butanoate) (compoundVIII")

6-iso-bendamustine ethylester having the structural formula 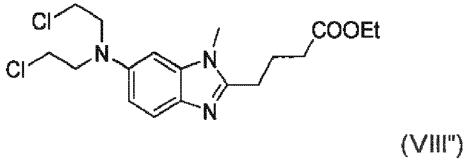 was prepared as follows:

A round bottom flask equipped with a magnetic stirring bar and a reflux-condenser with an oil valve was charged with phosphorus oxychloride (2.0 ml, 21.9 mmol) and heated to an internal temperature of 65°C. Ethyl 4-{6-[bis(2-hydroxyethyl)amino]-1-methy)-1H-benzimidazol-2-yl}butanoate (1.0 g, 2.9 mmol) was added in portions within 13 min. After the addition was completed, the mixture was heated to reflux temperature and stirring was continued for a further 10 min. The mixture was allowed to reach room temperature and 1,2-dimethoxyethane (2.3 ml) was added with stirring.

A second round bottom flask was charged with potassium bicarbonate (10.7 g, 107 mmol) and potable water (13 ml). The product solution was added slowly with stirring to the bicarbonate within 20 min, maintaining an internal temperature of about 18 to 28°C. The suspension was diluted with potable water (10 ml), and 6-iso-bendamustine ethylester was isolated as a solid by means of filtration, washed with potable water (4 x 2.5 ml) and used in the next synthetic step without further purification.

Yield (moist): 1.73 g
¹H NMR (500 MHz, DMSO-d₆, ppm): δ = 7.37 (d, ³J=8.7 Hz, 1H, arom. H-4), 6.78 (expected ⁴J coupling not resolved, 1 H, arom. H-7), 6.68 (d, ³J=8.7 Hz, expected ⁴J coupling not resolved, 1H, arom. H-5), 4.05 (q, ³J=7.0 Hz, 2H, OCH₂CH₃), 3.75 (s, 8H, CH₂CH₂Cl), 3.65 (s, 3H, CH₃N), 2.82 (t, ³J = 7.3 Hz, 2H, CH₂-CH₂-CH₂-Ester), 2.44 (t, ³J = 7.2 Hz, 2H, CH₂-CH₂-CH₂-Ester), 1.99 (m, 2H, CH₂-CH₂-CH₂-Ester), 1.18 (t, ³J=7.0 Hz, 3H, OCH₂CH₃).
¹³C-{¹H}NMR (125 MHz, DMSO-d₆, ppm): δ = 173.1 (COOEt), 153.3 (arom. CN₂), 143.0 (arom. CN(CH₂CH₂Cl)₂), 137.6 (arom.), 135.5 (arom.), 119.3 (arom. C-4), 109.3 (arom. C-5), 94.0 (arom. C-7), 60.2 (CH₂CH₃), 53.7 (N(CH₂CH₂Cl)₂), 42.0 (N(CH₂CH₂Cl)₂), 33.3 (CH₂-CH₂-CH₂-Ester), 29.8 (CH₃), 26.1 (CH₂-CH₂-CH₂-Ester), 22.6 (CH₂-CH₂-CH₂-Ester), 14.6 (CH₂,CH₃).
HPLC-purity: 94.1% relative area
LC-MS (ESI⁺): m/z = 386.0 (M + H⁺; 100% relative intensity)

### Example 8: 6-iso-bendamustine hydrochloride containing about 3.8 wt.-% of water (IUPAC-nomenclature: 4-{6-[bis(2-chloroethyl)amino]-1-methyl-1H-benzimidazol-2-yl)butanoic acid hydrochloride) (compound IX'")

6-iso-bendamustine hydrochloride having the structural formula 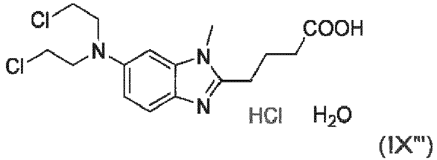 containing about 3.8 wt.-% of water was prepared as follows:

A round bottom flask was charged with the crude 6-iso-bendamustine ethyl ester (1.5 g, moist product) and hydrochloric acid (37%, 5.0 ml). The solution was concentrated with a rotary evaporator at 60 °C bath temperature under reduced pressure during 4 h. The viscous concentrate was allowed to cool down to about 38 °C and reverse osmosis water (10 ml) was added. Vigorous stirring of the mixture yielded an emulsion which solidified upon scratching the glass wall of the vessel. Stirring was continued for a further 15 min at ambient temperature and the precipitate was isolated by filtration, washed with reverse osmosis water (3 x 0.4 ml) to yield 6-iso-bendamustine hydrochloride in the form of a colorless solid containing about 3.8 wt.-% of water after drying over-night in a constant stream of air.

Yield: 0.54 g (46%)
¹H NMR (500 MHz, DMSO-d₆, ppm): δ = 16-14 (s, br, 0.7H, acidic), 13-11 (s, br, 0.7H, acidic), 7.57 (d, ³J=9.1 Hz, 1 H, arom. H-4), 7.11 (d, ⁴J=1.7 Hz, 1 H, arom. H-7), 7.07 (dd, ³J=9.1 Hz, ⁴J=1.7 Hz, 1 H, arom. H-5), 3.90 (s, 3H, CH₃N), 3.86 (t, ³J= 6.7 Hz, 4H, 2x NCH₂CH₂Cl), 3.80 (t, ³J = 6.7 Hz, 4H, NCH₂CH₂Cl), 3.16 (t, ³J = 7.6 Hz, 2H, CH₂-CH₂-CH₂-COOH), 2.41 (t, ³J =7.2 Hz, 2H, CH₂-CH₂-CH₂-COOH), 2.02 (m, 2H, CH₂-CH₂-CH₂-COOH); acidic protons diminished in intensity, presumably due to solvent exchange. ¹³C-{¹H}-NMR (125 MHz, DMSO-d₆, ppm): δ = 174.1 (COOH), 152.1 (arom. CN₂), 145.8 (arom. CN(CH₂CH₂Cl)₂), 134.7 (arom.), 122.3 (arom.), 115.0 (arom. C-4), 113.1 (arom. C-5), 94.5 (arom. C-7), 53.0 (N(CH₂CH₂Cl)₂), 41.7 (N(CH₂CH₂Cl)₂), 33.0 (CH₂-CH₂-CH₂-COOH), 31.3 (CH₃), 24.0 (CH₂-CH₂-CH₂-COOH), 21.7 (CH₂-CH₂-CH₂-COOH).
IR (KBr): see spectrum; wavenumber=1719 (COOH)
Water content by Karl-Fischer-titration: 3.8% (calculated water content for 6-iso-bendamustine hydrochloride containing one molecule of water per molecule: 4.4%) Chloride content by titration: 8.5% (calculated chloride content for mono-hydrochloride: 8.6%)
HPLC-purity: 98.6% relative area
NMR-purity: >99% (calculated for 6-iso-bendamustine HCl H₂O)
LC-MS (ESI⁺): see TIC and spectrum; m/z = 358.1 (M - Cl⁻- H₂O; 100% relative intensity) Melting point (not corrected): 174 - 176.5 °C (conducted manually with a melting point microscope; intermediate melting/re-crystallization was not visible)
DSC (heating rate: 10 K/min): first melting peak at 112.4 °C (onset: 106.1°C); a recrystallization minimum at 119.8°C(onset: 117.1°C); a second melting peak at 187.7 °C (onset:185.2°C)

Powder X-ray diffraction (XRD) data of the ten most intense signals (2θ and D values rounded to the second decimal place, I/I₀ values are rounded to first decimal place):

| entry | **2θ [°]** | **D [Å⁻¹]** | **I/I₀** |
|---|---|---|---|
| 1 | 7.83 | 11.29 | 231.7 |
| 2 | 10.01 | 8.83 | 157.3 |
| 3 | 13.18 | 6.71 | 94.9 |
| 4 | 13.75 | 6.43 | 97.0 |
| 5 | 14.96 | 5.92 | 161.3 |
| 6 | 15.34 | 5.77 | 150.0 |
| 7 | 18.47 | 4.80 | 397.7 |
| 8 | 22.27 | 3.99 | 1000.0 |
| 9 | 24.63 | 3.61 | 301.8 |
| 10 | 26.53 | 3.36 | 268.7 |

### Example 9: Determination of the half-lives of bendamustine and iso-bendamustine derivatives according to the present invention

The half-lives of bendamustine and iso-bendamustine (each in form of the free acid and as an ethylester) were determined in phosphate buffer (pH = 7.2) at 37°C

| compound | half-life [minutes] |
|---|---|
| bendamustine-HCl | 10.8 ± 2.9 |
| bendamustine ethyl ester | 8.9 ± 1.4 |
| iso-bendamustine-HCl | 11.6 ± 1.9 |
| iso-bendamusatine ethyl ester | 15.3 ± 3.8 |

From the results it can be derived that iso-bendamustine has a somewhat prolonged half-life as compared to its bendamustine counterpart. The half-life of the iso-bendamustine ethy lester is significantly increased as compared to the corresponding free acid and almost doubled as compared to the bendamustine ethyl ester.

Furthermore, the half-lives (in minutes) of bendamustine and iso-bendamustine (each in form of the free acid and as an ethylester) were also determined in murine and human plasma at 37°C.

| compound | murine plasma | human plasma |
|---|---|---|
| bendamustine-HCl | 35 ± 2.2 | 131 ± 10.2 |
| bendamustine ethyl ester | < 2 | 116.6 ± 9 |
| iso-bendamustine-HCl | 60.5 ± 2.7 | 83.8 ± 9.3 |
| iso-bendamustine ethyl ester | < 2 | 140.9 ± 6.3 |

The results obtained confirm the longest half-life for the iso-bendamustine ethyl ester which is much increased as compared to the corresponding free acid.

### Example 10: Determination of the IC50 values [µM] of bendamustine and iso-bendamustine derivatives according to the present invention

The **IC50** values [µM] of bendamustine and iso-bendamustine (each in form of the free acid and as an ethylester) were determined on various tumor cell lines after an incubation period of 96 hours.

| **Cell line** | **bendamustine-HCl** | **bendamustine ethyl ester** | **iso-benda-mustine-HCl** | **iso-benda-mustine ethyl ester** |
|---|---|---|---|---|
| SK-Mel3 ^{a} | > 100 | 12.4 ± 2.7 | > 100 | 11.7 ± 2.5 |
| HEL 92.1.7 ^{b} | 84.0 ± 7.7 | 2.9 ± 0.2 | 76.5 ± 4.5 | 3.5 ± 0.3 |
| Jurkat ^{b} | 42.6 ± 5.6 | 3.8 ± 0.15 | 59.5 ± 4.0 | 3.9 ± 0.3 |
| U-937 ^{b} | 91.9 ± 16.4 | 1.9 ± 0.04 | 59.8 ± 5.2 | 1.6 ± 0.03 |
| Capan-1^{a} | 25.2 ± 5.4 | 0.91 ± 0.11 | 24.6 ± 4.4 | 0.91 ± 0.15 |
| LnCap clone FGC ^{b} | 71.6 ± 5.4 | 2.04 ± 0.42 | 18.9 ± 2.6 | 1.5 ± 0.34 |
| NCl-H460 ^{a} | 89.5 ± 10.4 | 4.8 ± 0.2 | > 100 | 5.4 ± 0.2 |
| HT-29 ^{a} | > 100 | 10.1 ± 0.5 | > 100 | 11 ± 1.1 |
| MG-63 ^{a} | 55 ± 3.3 | 2.1 ± 0.05 | 50.1 ± 5.5 | 1.9 ± 0.1 |
| SK-ES1 ^{b} | 11.0 ± 1.6 | 0.19 ± 0.09 | 9.6 ± 1.5 | 0.24 ± 0.04 |

| | | | | |
|---|---|---|---|---|
| ^{a} Investigated in the crystal violet assay (ref.: Bernhardt G, Reile H, Birnböck H, Spruss T, Schönenberger H. Standardized kinetic microassay to quantify differential chemosensitivity on the basis of proliferative activity. J. Cancer Res. Clin. Oncol. 1992, 118: 35-43) ^{b} Investigated in the MTT assay (ref.: Mosmann T; Rapid colorimetric assay for cellular growth and survival: application to proliferation and cytotoxicity assay; J. Immunol. Methods 1983, 65: 55-63) | | | | |

The data clearly indicate that the iso-bendamustine ethyl ester has - in all tumor cell lines tested - much improved IC50 values as compared to the corresponding free acid.

## Claims

1. A compound of formula IX or a salt thereof, 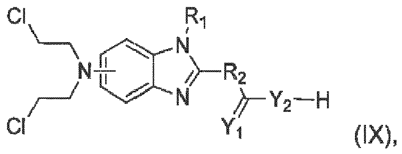 wherein the bis(2-chloroethyl)amino group is attached to position 6 of the benzimidazole ring structure; and
wherein R₁ is alkyl, aryl or alkylaryl; R₂ is alkanediyl, arylene, alkylarylene or arylalkanediyl; and Y₁ and Y₂ are independently from each other oxygen or sulfur.

2. The compound of claim 1, wherein compound of formula IX is in the form of a base addition salt in which the base is selected from the group consisting of magnesium hydroxide, calcium hydroxide, zinc hydroxide, potassium hydroxide, sodium hydroxide, potassium carbonate, sodium carbonate, potassium hydrogen carbonate, sodium hydrogen carbonate, diethylamine, triethylamine, ethanolamine (2-aminoethanol), diethanolamine (2,2'-iminobis(ethanol)), triethanolamine (2,2',2"-nitrilotris(ethanol)), ethylenediamine, piperazine, piperidine, pyrrolidine, pyridine, morpholine, 1H-imidazole, N-methyl-glucamine, L-lysine, L-arginine, benethamine, choline, 4-(2-hydroxyethyl)-morpholine, tromethamine, 2-(dimethylamino)ethanol (Deanol), 1-(2-hydroxyethyl)-pyrrolidine, 2-(diethylamino)ethanol, benzathine, hydrabamine, and betaine.

3. The compound of claim 1 or 2, wherein compound of formula IX is in the form of a salt, preferably a salt comprising 0.6 to 1.4 mol water relative to 1 mol of compound of formula IX, more preferably 0.8 to 1.2 mol water, even more preferably the salt comprising water is in the form of a hydrate.

4. A compound of formula VIII or a salt thereof, 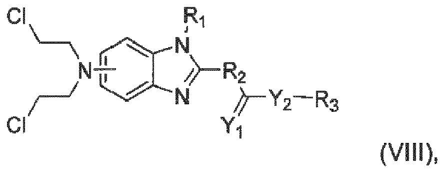 wherein the bis(2-chloroethyl)amino group is attached to position 6 of the benzimidazole ring structure;
wherein R₁ and R₃ are independently from each other alkyl, aryl or alkylaryl; R₂ is alkanediyl, arylene, alkylarylene or arylalkanediyl; Y₁ and Y₂ are independently from each other oxygen or sulfur; and
wherein optionally R₃ is substituted with an amine moiety -NR₅R₆ in which R₅ and R₆ independently from each other are a substituted or unsubstituted alkyl, or R₅ and R₆ together represent a C₃-C₇ alkyl chain forming a 4 to 8-membered ring structure together with the nitrogen located between R₅ and R₆, wherein one or more carbon atoms in said ring structure are optionally replaced by heteroatoms selected from the group consisting of nitrogen, oxygen or sulfur.

5. A compound of formula VII or an acid addition salt thereof, 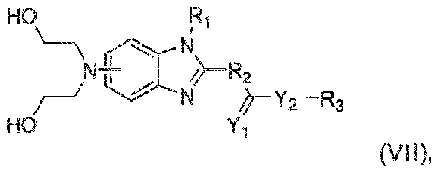 wherein the bis(2-chloroethyl)amino group is attached to position 6 of the benzimidazole ring structure;
wherein R₁ and R₃ are independently from each other alkyl, aryl or alkylaryl; R₂ is alkanediyl, arylene, alkylarylene or arylalkanediyl; Y₁ and Y₂ are independently from each other oxygen or sulfur; and
whwerein optionally R₃ is substituted with an amine moiety -NR₅R₆ in which R₅ and R₆ independently from each other are a substituted or unsubstituted alkyl, or R₅ and R₆ together represent a C₃-C₇ alkyl chain forming a 4 to 8-membered ring structure together with the nitrogen located between R₅ and R₆, wherein one or more carbon atoms in said ring structure are optionally replaced by heteroatoms selected from the group consisting of nitrogen, oxygen or sulfur.

6. The compound of any one of claims 1 to 5, **characterized by** at least one of the following structural features:
(a) R₁ is C₁-C₆ alkyl, R₂ is C₁-C₆ alkanediyl, and Y₁ and Y₂ are oxygen; wherein preferably R₁ is C₁-C₄ alkyl, R₂ is C₁-C₄ alkanediyl, and Y₁ and Y₂ are oxygen;
(b) R₁ is C₁-C₃ alkyl, R₂ is C₁-C₃ alkanediyl, and Y₁ and Y₂ are oxygen;
(c) R₁ is methyl, R₂ is propanediyl, and Y₁ and Y₂ are oxygen; and
(d) in compounds of formula VIII and VII, R₃ is C₁-C₃ alkyl, and preferably ethyl.

7. The compound of formula VIII or VII of any one of claims 4 to 6, wherein R₃ is substituted with an amine moiety -NR₅R₆ in the form of a ring structure formed by R₅ and R₆ together with the nitrogen located between R₅ and R₆, wherein said ring structure is **characterized by** at least one of structural features:
(i) one carbon atom is replaced by one nitrogen atom or one oxygen atom, preferably by one oxygen atom;
(ii) a further nitrogen atom is substituted (-NR₇-) or unsubstituted (-NH-), preferably substituted with R₇ being selected from the group consisting of alkyl, aryl, alkylaryl or arylalkyl, more preferably with alkyl;
(iii) the ring structure is in the form of a 5 or 6-membered ring, preferably a 6-membered ring;
(iv) the ring structure is selected from the group consisting of pyrrolidine, piperidine, piperazine and morpholino, preferably being piperazine or morpholino, and more preferably being morpholino;
(v) the atoms of the ring structure are unsubstituted, or substituted with a substituent selected from the group consisting of C₁-C₄ alkyl, C₁-C₄ alkoxy, C₁-C₄ alkylsulfide, unsubstituted amino (-NH₂), dialkylamino in which alkyl is C₁-C₄ alkyl; the substituent preferably being C₁-C₄ alkyl or C₁-C₄ alkoxy; and more preferably the ring structure being unsubstituted.

8. The compound of any one of claims 1 to 7, wherein compound is in the form of an acid addition salt in which the acid is selected from the group consisting of hydrochloric acid, hydrobromic acid, sulfuric acid, phosphoric acid, glutamic acid, (+)-L-tartaric acid, citric acid, (-)-L-malic acid, DL-lactic acid, L-ascorbic acid, succinic acid, adipic acid, acetic acid, stearic acid, carbonic acid, thiocyanic acid, glycerol-phosphoric acid, L-aspartic acid, maleic acid, fumaric acid, galactaric acid, D-glucuronic acid, glycolic acid, D-glucoheptonic acid, hippuric acid, D-gluconic acid, glutaric acid, sebacic acid, capric (decanoic) acid, lauric acid, palmitic acid, alginic acid, benzoic acid, nicotinic acid, propionic acid, caprylic (octanoic) acid, naphthalene-1,5-disulfonic acid, ethane-1,2-disulfonic acid, cyclamic acid, p-toluenesulfonic acid ,methanesulfonic acid, dodecylsulfuric acid, naphthalene-2-sulfonic acid, benzenesulfonic acid, oxalic acid, 2-hydroxy ethanesulfonic acid, ethanesulfonic acid, pannoic (embonic) acid, 2-oxoglutaric acid, 1-hydroxy-2-naphthoic acid, malonic acid, gentisic acid, lactobionic acid, (-)-L-pyroglutamic acid ,oleic acid, (+)-camphoric acid, isobutyric acid, and orotic acid.

9. The compound of formula IX and VIII of any one of claims 1 to 4 and 6 to 8, being in the form of the free acid/base or a pharmaceutically acceptable salt thereof, for use in the therapeutic treatment of a disease being selected from the group consisting of acute T cell leukemia, erythroleukemia, Ewing osteosarcoma, (hormone dependent) mamma carcinoma, cervix carcinoma, colorectal cancer, medulloblastoma, glioblastoma and astrocytoma, malignant melanoma, histocytic lymphoma, pancreatic carcinoma, prostate cancer (metastasis of a subclavicular lymph node), large cell bronchial carcinoma, colorectal adenocarcinoma, and osteosarcoma; wherein the disease is preferably selected from the group consisting of acute T cell leukaemia, erythroleukemia, Ewing osteosarcoma, malignant melanoma, histocytic lymphoma, pancreatic carcinoma, prostate cancer (metastasis of a subclavicular lymph node), large cell bronchial carcinoma, colorectal adenocarcinoma, and osteosarcoma; and more preferably selected from the group consisting of Ewing osteosarcoma, malignant melanoma, pancreatic carcinoma, prostate cancer (metastasis of a subclavicular lymph node), colorectal adenocarcinoma, and osteosarcoma.

10. A process for preparing a compound of formula V or an acid addition salt thereof, 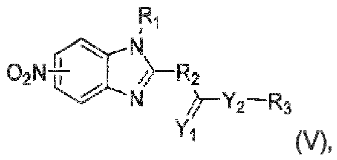 wherein the nitro group is attached to position 6 of the benzimidazole ring structure; and
wherein R₁ is alkyl, aryl or alkylaryl; R₂ is alkanediyl, arylene, alkylarylene or arylalkanediyl; R₃ is H, alkyl, aryl or alkylaryl; and Y₁ and Y₂ independently from each other are oxygen or sulfur;
the process comprising:
(i) when R₃ is alkyl, aryl or alkylaryl, reacting a compound of formula II 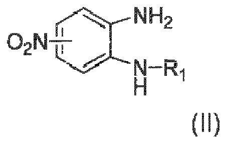 wherein R₁ is defined as above, and the nitro group is attached to any one of positions 3, 4 and 6 of the aniline moiety,
with a compound of formula III¹ 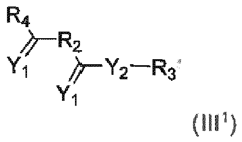 wherein R₂, Y₁ and Y₂ are defined as above, R₃' is alkyl, aryl or alkylaryl, and R₄ is selected from the group consisting of -Y₂-H, -Cl and -Y₂-CY₁-R₂-CY₁-Y₂-R₃'; or
(ii) when R₃ represents H, reacting a compound of formula II with a compound of formula III² or a compound of formula III³ 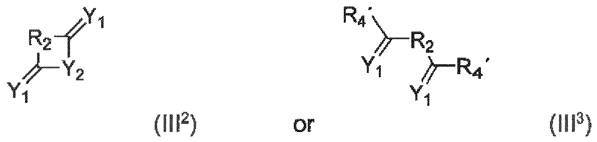 wherein R₂, Y₁ and Y₂ are defined as above, and the two substituents R₄' in formula III³ independently from each other are -Y₂-H or -Cl, preferably both R₄' represent -Y₂-H or one R₄' = -Y₂-H and the other R₄' = -Cl,
with the proviso that in the case compound of formula III³ with both R₄' = -Cl, the chloro group not forming an amide with the amine moiety of formula II is hydrolized for converting said chloro group to a hydroxy group.

11. The process of claim 10, wherein said process is carried out without isolation of an intermediate compound of formula IV or an acid addition salt thereof 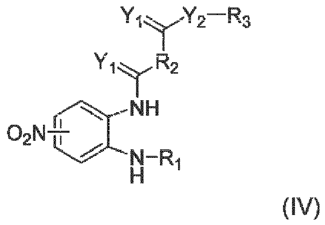 wherein R₁ is alkyl, aryl or alkylaryl; R₂ is alkanediyl, arylene, alkylarylene or arylalkanediyl; R₃ is H, alkyl, aryl or alkylaryl; and Y₁ and Y₂ independently from each other are oxygen or sulfur.

12. The process of claim 10 or 11, wherein R₁ is C₁-C₃ alkyl, R₂ is C₁-C₃ alkanediyl, R₃ is H or C₁-C₃ alkyl, and Y₁ and Y₂ are oxygen; and wherein preferably R₁ is methyl, R₂ is propanediyl, R₃ is H or ethyl and Y₁ and Y₂ are oxygen; and/or in the compound of formula III¹, III² and III³, Y₁ and Y₂ are oxygen; and/or in the compound of formula III¹, R₄ = Y₂-CY₁-R₂-CY₁-Y₂-R₃'.

13. A process for preparing a compound of formula IX, VIII or VII of any one of claims 1 to 9, comprising the steps of:
(a) preparing a compound of formula V as specified in any one of claims 10 to 12, and
(b) further converting the compound of formula V to any one of compounds of formula IX, VIII or VII.

14. A pharmaceutical composition comprising a compound of formula IX and/or VIII as specified in any one of claims 1 to 4 and 6 to 9, being in the form of the free acid/base or a pharmaceutically acceptable salt thereof, as pharmaceutically active agent(s) and at least one pharmaceutically acceptable excipient.

15. The pharmaceutical composition of claim 14 for oral and/or parenteral administration.
